# EUROPÄISCHE PATENTSCHRIFT

(11) **EP 3 077 250 B1**
(45) Veröffentlichungstag und Bekanntmachung des Hinweises auf die Patenterteilung: **21.03.2018**
(21) Anmeldenummer: 13807951.2
(22) Anmeldetag: 04.12.2013
(51) Int. Cl.: A61L 2/18, B08B 3/08, B08B 3/10, G21F 9/00, B60P 3/00, E04H 1/12

(54) **GERÄTESYSTEM FÜR MILITÄRISCHE UND/ODER HUMANITÄRE OPERATIONEN, INSBESONDERE MOBILES DEKONTAMINATIONSSYSTEM**
DEVICE SYSTEM FOR MILITARY AND/OR HUMANITARIAN OPERATIONS, IN PARTICULAR A MOBILE DECONTAMINATION SYSTEM
SYSTÈME D'APPAREILS POUR OPÉRATIONS MILITAIRES ET/OU HUMANITAIRES, NOTAMMENT SYSTÈME DE DÉCONTAMINATION MOBILE

(43) Veröffentlichungstag der Anmeldung: 12.10.2016
(73) Patentinhaber: Kärcher Futuretech GmbH, 71409 Schwaikheim (DE)
(72) Erfinder: POPP, Thomas, 70174 Stuttgart (DE); REINHARDT, Ulli, 71522 Backnang (DE); RUPRECHT, Daniel, 70734 Fellbach (DE)
(74) Vertreter: Witte, Weller & Partner Patentanwälte mbB
(86) Internationale Anmeldenummer: PCT/EP2013/075581
(87) Internationale Veröffentlichungsnummer: WO 2015/081998

(56) Entgegenhaltungen:
- EP-A2- 2 617 630
- WO-A1-2006/000795
- BE-A- 701 367
- DE-A1- 19 653 731

## Beschreibung

Die vorliegende Erfindung betrifft ein Gerätesystem für militärische und/oder humanitäre Operationen, insbesondere ein mobiles Dekontaminationssystem, mit einer Vielzahl an elektrischen und/oder kraftstoffbetriebenen Aggregaten, Zubehörteilen und Betriebsstoffen, die zusammen einen Funktionsumfang des Gerätesystems bestimmen, wobei die elektrischen und/oder kraftstoffbetriebenen Aggregate zumindest eine der folgenden Komponenten beinhalten: einen Generator zur Erzeugung von elektrischem Strom, eine oder mehrere Pumpen zur Umwälzung, Förderung und/oder Austragung von Flüssigkeiten, eine oder mehrere Pumpen zur Erzeugung von Unterdruck, einen Brenner und/oder eine elektrische Heizung zur Erhitzung von auszutragenden Flüssigkeiten, Dekontaminationsmitteln und/oder anderen Chemikalien, Filter, Prozesssteuergeräte und ein Bedienpult, ferner mit einer lasttragenden Grundplatte mit einer definierten Stellfläche und mit Ankerelementen, die eine lösbare Verankerung an einem Transportmittel, insbesondere an einem Transportfahrzeug, ermöglichen, und mit einer Haltekonstruktion, die auf der Stellfläche befestigt ist und die dazu ausgebildet ist, die Aggregate beim Transport und im Betrieb auf der Grundplatte zu halten.

DE 196 53 731 A1 offenbart eine mobile Zapfanlage zur Befüllung eines mobilen Gastanks mit Merkmalen gemäß dem Oberbegriff von Anspruch 1. In einer Ausführungsform sind jeweils zwölf Druckgasflaschen in einem korbartigen Gestell angeordnet. Die so gebildeten Batterien mit Druckgasflaschen sind zusammen mit einer Verdichteranlage, einer Zapfsäule und diversen Nebenaggregaten in einem Container angeordnet, der auf einem Lastkraftwagen befestigt ist.

Ein solches Gerätesystem ist ferner das Dekontaminationssystem DECOCONTAIN 3000 GDS der Kärcher Futuretech GmbH mit Sitz in 71409 Schwaikheim, Deutschland. Dieses Dekontaminationssystem ist ein typisches Beispiel für ein Gerätesystem für militärische oder auch humanitäre Operationen. Es beinhaltet eine Vielzahl von Aggregaten, wie etwa eine oder mehrere Pumpen, einen Aerosolgenerator, Heizgeräte und Steuergeräte, sowie eine Vielzahl von Zubehörteilen, wie etwa Sprühlanzen oder Schläuche, und eine Vielzahl von Betriebsstoffen, wie insbesondere Dekontaminationsmittel. Die Aggregate, Zubehörteile und Betriebsstoffe bestimmen und begrenzen den Funktionsumfang, der typischerweise von den Käufern und Anwender eines solchen Systems im Rahmen einer Ausschreibung und/oder anhand eines Forderungskatalogs definiert wird. Die Gesamtheit der Aggregate, Zubehörteile und Betriebsstoffe ist bei solchen Systemen in aller Regel so schwer, dass die Systeme nur mittels geeigneter Transportmittel, wie etwa einem Lastkraftwagen oder einem Hubschrauber zum Einsatzort gebracht werden können. Sehr häufig werden solche Gerätesysteme in Containern aufgebaut, die sich an der ISO-Norm 668 für Seefrachtcontainer orientieren. Die Container werden dann an den Transportfahrzeugen verankert. Die Nutzung der Gerätesysteme erfolgt häufig aus dem Container und von dem Transportfahrzeug aus, ist prinzipiell jedoch auch abgesetzt vom Transportfahrzeug denkbar.

Eine Herausforderung für den Hersteller solcher Gerätesysteme liegt darin, einen vom Anwender geforderten Funktionsumfang innerhalb eines ebenfalls vom Anwender spezifizierten Raumvolumens unterzubringen. Beispielsweise kann das Raumvolumen von den Abmessungen und Gewichten abhängen, die ein bestimmtes Transportfahrzeug maximal transportieren kann. In der Regel sind die Gerätesysteme an die speziellen Anforderungen des Anwenders angepasst, was einen hohen Entwicklungs- und Herstellungsaufwand bei vergleichsweise kleinen Stückzahlen zur Folge hat. Gerade die optimale Raumplanung für ein neues Gerätesystem stellt den Hersteller immer wieder vor große Herausforderungen.

Das eingangs genannte Dekontaminationssystem DECOCONTAIN 3000 GDS ist in einem 20 Fuß ISO-Container aufgebaut. Innerhalb des Containers sind mehrere Stützpfeiler befestigt, die eine Haltekonstruktion für die schweren Aggregate bilden. Die Position der einzelnen Stützpfeiler innerhalb des Containers hat sich aus der räumlichen Anordnung der Aggregate, Zubehörteile und Betriebsstoffe innerhalb es Containers und aus statischen Überlegungen ergeben. Wenn ein Anwender einen geänderten Funktionsumfang haben möchte, weil er beispielsweise eine größere Anzahl an temperaturempfindlichen Kleinteilen pro Stunde dekontaminieren möchte, erfordert dies häufig eine grundlegende Neukonstruktion des Systems.

DE 103 45 351 A1 beschreibt ein mobiles Dekontaminationssystem, bei dem funktionsbestimmende Aggregate, Zubehörteile und Betriebsstoffe in transportablen Containern untergebracht sind. Die Details der Haltekonstruktion in den Containern sind in den Figuren nur zum Teil sichtbar. Im Prinzip handelt es sich jedoch auch hier um ein Gerätesystem, bei dem die räumliche Anordnung der einzelnen Komponenten in den Containern in Abhängigkeit von dem vorgegebenen Funktionsumfang individuell entwickelt wurde.

Weitere mobile Dekontaminationssysteme sind aus US 2010/0299826 A1 und WO 03/046314 A1 bekannt. Auch diese System besitzen einen Container, in dem die funktionsbestimmenden Komponenten individuell untergebracht sind. WO2006/000795A1 und EP2617630A2 offenbaren Rahmengestelle für mobile Gerätesysteme gemäß dem Stand der Technik.

Ausgehend von diesem Stand der Technik ist es eine Aufgabe der vorliegenden Erfindung, ein Gerätesystem der eingangs genannten Art anzugeben, das auf effiziente Weise eine Anpassung an individuelle Anforderungen eines Anwenders ermöglicht. Es ist eine weitere Aufgabe der Erfindung, ein Gerätesystem der eingangs genannten Art anzugeben, welches der Hersteller auf einfache und effiziente Weise an individuelle Anforderungen eines Anwenders anpassen kann. Eine weitere Aufgabe der Erfindung besteht darin, ein Gerätesystem der eingangs genannten Art anzugeben, welches auf kostengünstige Weise mit unterschiedlichem Funktionsumfang realisiert werden kann.

Die Lösung dieser Aufgabe wird durch die im Anspruch 1 angegebenen Merkmale erhalten. Gemäß einem Aspekt der vorliegenden Erfindung werden diese Aufgaben durch ein Gerätesystem der eingangs genannten Art gelöst, bei dem die Haltekonstruktion aus einer Vielzahl von selbsttragenden, strukturgleichen und jeweils quaderförmigen Rahmengestellten gebildet ist, die nebeneinander und/oder aufeinander angeordnet und an der Grundplatte befestigt sind, wobei die Rahmengestelle jeweils achtvorzugsweise gleiche - Eckstücke und zwölf Kantenprofile besitzen, die zusammen jeweils ein definiertes Lagervolumen umgeben, und wobei die Aggregate, Zubehörteile und Betriebsstoffe durchweg in den definierten Lagervolumina der Rahmengestelle angeordnet sind.

Das neue Gerätesystem besitzt aufgrund der Rahmengestelle einen modularen und daher recht flexiblen Aufbau. Jedes Rahmengestell bildet ein definiertes Lagervolumen, in dem ein oder mehrere Aggregate, Zubehörteile und/oder Betriebsstoffe untergebracht werden können. In den bevorzugten Ausführungsbeispielen sind die Aggregate, Zubehörteile und Betriebsstoffe des neuen Gerätesystems ausschließlich in den definierten Lagervolumina der Rahmengestelle angeordnet. Abweichend hiervon ist es jedoch in anderen Ausführungsbeispielen denkbar, dass einzelne Aggregate, Zubehörteile und Betriebsstoffe des Gerätesystems außerhalb eines Rahmengestells angeordnet sind, beispielsweise weil es sich um ein sehr großes Aggregat oder um sehr unförmige Zubehörteile handelt. In diesen Fällen sind jedoch zumindest weitere Aggregate sowie Zubehörteile und Betriebsstoffe in den definierten Lagervolumina der Rahmengestelle angeordnet. Das neue Gerätesystem besitzt somit eine Vielzahl von Rahmengestellen, die die funktionsbestimmenden Aggregate, Zubehörteile und Betriebsstoffe zumindest überwiegend und vorzugsweise ausschließlich aufnehmen. Dies gilt sowohl für den Transport als auch für den Betrieb des Gerätesystems am Einsatzort.

Die selbsttragenden Rahmengestelle sind jeweils so stabil ausgebildet, dass sie die integrierten Aggregate, Zubehörteile und/oder Betriebsstoffe nach Art eines Kleincontainers umhäusen und tragen können. Dementsprechend ist es bei dem neuen Gerätesystem prinzipiell möglich, die Rahmengestelle mit den Aggregaten, Zubehörteilen und/oder Betriebsstoffen einzeln oder in ihrer Gesamtheit von der lasttragenden Grundplatte herunterzunehmen. In einigen Ausführungsbeispielen besitzen die Rahmengestelle Halteösen zum Befestigen eines Kranseils und/oder definierte Öffnungen, in die die Gabel eines Gabelstaplers eingreifen kann. Die Halteösen und/oder Öffnungen erleichtern die Montage des neuen Gerätesystems beim Hersteller und sie erleichtern auch den Austausch, die Wartung und die Reparatur defekter Komponenten.

Darüber hinaus bilden die selbsttragenden, strukturgleichen Rahmengestelle eine Art Baukastensystem mit quaderförmigen "Blöcken", die innerhalb eines Containers oder auch auf einer offenen lasttragenden Grundplatte sehr einfach zu einem Gesamtsystem kombiniert werden können. Die Unterbringung der Aggregate, Zubehörteile und Betriebsstoffe in den Rahmengestellen erleichtert es daher dem Hersteller, ein neues Gerätesystem nach individuellen Anforderungen eines Anwenders zusammenzustellen. Des Weiteren entfällt der bisher erforderliche Aufwand, eine Haltekonstruktion für die Aggregate, Zubehörteile und Betriebsstoffe an die individuellen Anforderungen des Anwenders anzupassen und gegebenenfalls in Sonderanforderung herzustellen. Da die Rahmengestelle zueinander strukturgleich sind und vorzugsweise gleiche Eckstücke besitzen, ergeben sich im Vergleich zu bisherigen Gerätesystemen günstigere Herstellungskosten für die Haltekonstruktion, selbst wenn bei dem neuen Gerätesystem "unnötige" Ecken und Kantenprofile verbaut werden.

Insgesamt erleichtert das neue Gerätesystem daher die Planung, Konstruktion und Herstellung eines an individuelle Anforderungen angepassten Gerätesystems für militärische und/oder humanitäre Operationen. Die oben genannten Aufgaben sind daher vollständig gelöst.

In einer bevorzugten Ausgestaltung belegen die Rahmengestelle einen zentralen Bereich der Stellfläche, so dass auf der Grundplatte an zumindest zwei Seiten, vorzugsweise an zumindest drei Seiten, ein freier Randbereich verbleibt.

In dieser Ausgestaltung ist die Haltekonstruktion mit den Rahmengestellen gewissermaßen das Zentrum des Gerätesystems auf der Grundplatte. Alternativ hierzu ist es in anderen Ausgestaltungen denkbar, die Rahmengestelle im Randbereich der Grundplatte so anzuordnen, dass der zentrale Bereich der Stellfläche frei bleibt. Die bevorzugte Ausgestaltung besitzt den Vorteil, dass der Randbereich relativ einfach mit Hilfe von Platten, Klappen oder dergleichen vergrößert werden kann, wenn das Gerätesystem am Einsatzort in Betrieb genommen werden soll. Die bevorzugte Ausgestaltung erleichtert daher die Nutzung des neuen Gerätesystems und sie ermöglicht eine sehr effiziente Nutzung des Ladevolumens von handelsüblichen 20 Fuß- oder 40 Fuß-Containern gemäß ISO 668.

In einer weiteren Ausgestaltung besitzt das Gerätesystem zumindest eine mobile Arbeitsplattform, die auf Höhe der Grundplatte an dem Randbereich positionierbar ist.

In den bevorzugten Ausführungsbeispielen kann die mobile Arbeitsplattform an dem Randbereich der Grundplatte befestigt werden, beispielsweise in geeignet ausgebildete Aufnahmen an der Grundplatte eingehängt werden. In anderen Ausführungsbeispielen ist die mobile Arbeitsplattform die Seitenwand eines Containers, die an der Grundplatte verschwenkbar gelagert ist und derart nach unten geklappt werden kann, dass sie auf Höhe der Grundplatte weitgehend parallel zur Grundplatte ausgerichtet werden kann. Die Ausgestaltung ermöglicht auf sehr einfache Weise einen im Betrieb vergrößerten Randbereich um die zentral auf der Grundplatte angeordneten Rahmengestelle. Daher eignet sich diese Ausgestaltung besonders gut in den Fällen, in denen die Grundplatte mit den Rahmengestellen auf einem Transportfahrzeug, wie etwa einem Lastkraftwagen, befestigt ist. Für den Transport kann die Arbeitsplattform abgenommen oder verschwenkt werden.

In einer weiteren Ausgestaltung sind die elektrischen und/oder kraftstoffbetriebenen Aggregate in den jeweiligen Rahmengestellen stationär montiert.

Der Betrieb der Aggregate erfolgt in dieser Ausgestaltung innerhalb der Rahmengestelle. Die stationäre Befestigung dieser Aggregate in den Rahmengestellen vereinfacht die Montage der Aggregate in den neuen Gerätesystemen und begünstigt einen störungsfreien Betrieb im Einsatz.

In einer weiteren Ausgestaltung weisen die Rahmengestelle ein einheitliches Rastermaß in Bezug auf Breite, Tiefe und/oder Höhe auf.

In dieser Ausgestaltung sind die Rahmengestelle nicht nur strukturgleich, sondern sie besitzen in zumindest einer Dimension und vorzugsweise in zumindest zwei Dimensionen ein ganzzahliges Vielfaches einer definierten Basisabmessung. In bevorzugten Ausführungsbeispielen besitzt das Gerätesystem eine Haltekonstruktion mit einer Vielzahl von Rahmengestellen, die sowohl in Bezug auf die Breite, Höhe und Tiefe gleiche Abmessungen aufweisen. Diese Ausgestaltungen ermöglichen eine besonders kostengünstige und flexible Planung, Konstruktion und Herstellung von Gerätesystemen, deren Funktionsumfang individuell an die Anforderungen eines Anwenders angepasst ist.

In einer weiteren Ausgestaltung beinhaltet die Vielzahl der Rahmengestelle erste Rahmengestelle und zweite Rahmengestelle, wobei die ersten Rahmengestelle eine erste Aufstandsfläche mit einer ersten Breite und einer ersten Tiefe sowie eine erste Höhe haben, wobei die zweiten Rahmengestelle eine zweite Aufstandsfläche mit einer zweiten Breite und einer zweiten Tiefe sowie eine zweite Höhe haben, wobei die zweite Tiefe gleich der ersten Tiefe ist, und wobei die zweite Höhe etwa ein Drittel der ersten Höhe ist. Vorteilhaft ist die zweite Breite das Doppelte der ersten Breite. In einigen Ausführungsbeispielen sind die erste und zweite Breite jedoch gleich. Bevorzugt besteht die gesamte Haltekonstruktion des Gerätesystems aus den ersten und zweiten Rahmengestellen.

In dieser Ausgestaltung können die ersten Rahmengestelle die halbe Breite der zweiten Rahmengestelle besitzen. Andererseits sind die ersten Rahmengestelle wesentlich höher als die zweiten Rahmengestelle. Die Haltekonstruktion wird somit einerseits aus schmalen, hohen Rahmengestellen und andererseits aus flachen und vorzugsweise breiten Rahmengestellen gebildet. Vorteilhaft haben alle Rahmengestelle dieselbe Tiefe. Zwei schmale erste Rahmengestelle passen mit der halben Breite exakt auf ein flaches zweites Rahmengestell. Die breiten flachen Rahmengestelle eignen sich hervorragend zur Aufnahme von Flüssigkeitstanks, da ein solcher Tank bei Befüllung sehr schwer ist und somit einen niedrigen Schwerpunkt des Gerätesystems begünstigt. Die schmalen, hohen Rahmengestelle dienen vorteilhaft zur Aufnahme der Aggregate, die in den höheren Rahmengestellen leichter montiert und bedient werden können. Aufeinander gestapelt lässt sich ein sehr flexibel konfigurierbares Gerätesystem mit einer sehr geringen Teilevielfalt für die Haltekonstruktion herstellen.

In einer weiteren Ausgestaltung beträgt die erste Breite etwa 90 cm und die erste Tiefe beträgt etwa 200 cm. Vorzugsweise ist die erste Höhe etwa 150 cm. Dabei bezeichnet die Angabe "etwa" hier einen Toleranzbereich von +/- 15 %.

Diese Maße für die ersten und zweiten Rahmengestelle haben sich nach vielen Erwägungen und Versuchen der Anmelderin als optimale Maße herausgestellt, um Gerätesysteme für militärische Operationen und insbesondere Dekontaminationssysteme zur Entstrahlung, Entseuchung und/oder Entgiftung von militärischen Ausrüstungsgegenständen zu konstruieren und herzustellen. Besonders vorteilhaft ist es, wenn das lichte Innenmaß der Rahmengestelle in der Breite mindestens 80cm beträgt. Diese Maße ermöglichen die Unterbringung von vielen verschiedenen Aggregaten. Sie begrenzen aufgrund des umspannten Lagervolumens dabei das maximale Gewicht eines einzelnen Moduls. Und sie machen es möglich, eine flexibel nutzbare Haltekonstruktion herstellen, die zum Transport mit zahlreichen militärischen Transportfahrzeugen und -fluggeräten geeignet ist.

In einer weiteren Ausgestaltung besitzt das Gerätesystem zumindest einen Flüssigkeitstank, der in einem der Rahmengestelle dauerhaft befestigt ist. Vorteilhafterweise ist das Rahmengestell ein flaches zweites Rahmengestell unter einem oder zwei hohen schmalen Rahmengestellen, wobei letztere auf dem flachen zweiten Rahmengestell angeordnet und befestigt sind.

Diese Ausgestaltung macht vorteilhaften Gebrauch von den bereits weiter oben angesprochenen Möglichkeiten, und sie hat sich insbesondere zur Realisierung eines Dekontaminationssystems bewährt, da sie einen leichten Zugang zu den Aggregaten in einer ergonomischen Arbeitshöhe und einen relativ niedrigen Schwerpunkt bei einem einsatzbereiten System ermöglicht.

In einer weiteren Ausgestaltung weisen die Eckstücke jeweils drei orthogonal zueinander angeordnete Zapfen auf, wobei jeder Zapfen mit jeweils einem Kantenprofil dauerhaft verbunden ist.

In bevorzugten Ausführungsbeispielen sind die Zapfen der Eckstücke mit dem jeweils verbundenen Kantenprofil verklebt, verschweißt und/oder verschraubt. In den bevorzugten Ausführungsbeispielen sind die Kantenprofile Gussprofile mit jeweils zumindest einer Hohlkammer, in die die Zapfen der Eckstücke hineinragen. Die Gussprofile sind bevorzugt mit einem Schwerkraftgussverfahren und insbesondere einem Kokillengussverfahren hergestellt, weil dies für die beabsichtigte Verwendung der Eckstücke das vorteilhafteste Herstellungsverfahren ist. Die Ausgestaltungen ermöglichen eine weitgehend glatte Außenkontur der Rahmengestelle, was in Bezug auf Reinigung und Raumausnutzung von Vorteil ist. Des Weiteren ist es in einigen Ausführungsbeispielen bevorzugt, wenn die Zapfen und Hohlkammern einen polygonalen Querschnitt haben, da dieser einem Verdrehen der Kantenprofile relativ zu den Zapfen entgegenwirkt. In bevorzugten Ausführungsbeispielen sind die Eckstücke Druckgussteile aus Aluminium. Des Weiteren ist es bevorzugt, wenn sämtliche Eckstücke der Rahmengestelle gleich sind, um die Teilevielfalt und Produktionskosten zu senken.

Es hat sich gezeigt, dass mit derartigen Eckstücken sehr stabile Rahmengestelle herstellbar sind, die aufgrund ihrer Stabilität sehr schwere Aggregate dauerhaft aufnehmen können. Die bevorzugte Ausbildung der Zapfen und Kantenprofile und deren dauerhafte Verbindung erleichtern einen Betrieb der Aggregate auch unter den rauen Einsatzbedingungen einer militärischen Operation.

In einer weiteren Ausgestaltung weisen die Eckstücke jeweils eine taschenförmige Vertiefung auf, die an einer Seite von einem U-förmigen Profil begrenzt ist, welches in der Vertiefung einen Hinterschnitt bildet.

Diese Ausgestaltung hat sich als eine optimale Variante erwiesen, um eine flexible und gleichzeitig robuste Verbindung zweier Rahmengestelle aneinander zu ermöglichen. Der Hinterschnitt in der taschenförmigen Vertiefung ermöglicht das Einsetzen von Steckbolzen, mit denen sich zwei Eckstücke auf sehr einfache und robuste Weise koppeln lassen. Die taschenförmige Vertiefung ermöglicht dabei große Materialstärken für den Steckbolzenkopf, was angesichts der hohen Kräfte, die die Rahmengestelle und insbesondere deren Eckstücke aufnehmen müssen, von Vorteil ist.

In einer weiteren Ausgestaltung weist das U-förmige Profil eine plane Außenseite auf, die eine definierte Überhöhung an dem zugehörigen Eckstück bildet.

In dieser Ausgestaltung bildet die plane Außenseite eine definierte Aufstandsfläche, die eine sehr stabile und lagegenaue Verbindung zwischen zwei aneinanderstoßen Eckstücken ermöglicht. In bevorzugten Ausführungsbeispielen ruhen die Rahmengestelle nur auf den definierten Aufstandsflächen von jeweils vier Eckstücken. Die U-förmigen Profile bilden dabei die höchste bzw. niedrigste Erhebung in der Außenkontur der Rahmengestelle, so dass das Gewicht der Rahmengestelle inklusive der darin angeordneten Aggregate, Zubehörteile und Betriebsstoffe auf den Eckstücken ruht. Diese Ausgestaltung vermeidet punktuelle Querbelastungen auf den Kantenprofilen und sie hat sich insbesondere zur Aufnahme von schweren Aggregaten oder Flüssigkeitstanks als eine sehr günstige Realisierung erwiesen.

In einer weiteren Ausgestaltung besitzt das Gerätesystem eine Vielzahl T-förmiger Steckbolzen, mit denen aneinander liegende Eckstücke von zwei übereinander angeordneten Rahmengestellen mechanisch verbunden sind.

In dieser Ausgestaltung sorgen T-förmigen Steckbolzen in vorteilhafter Weise für eine Fixierung der übereinander angeordneten Rahmengestelle in horizontaler Richtung. Dabei macht sich die Ausgestaltung die Schwerkraft zunutze. Zusätzlich besitzt das Gerätesystem in den bevorzugten Ausführungsbeispielen einen lösbaren Spannmechanismus, mit dessen Hilfe die übereinander angeordneten Rahmengestelle in vertikaler Richtung gegeneinander verspannt werden können. In einigen Ausführungsbeispielen besitzt der Spannmechanismus einen Exzenter, um eine hohe Spannkraft aufzubringen. In anderen Ausführungsbeispielen weist der Spannmechanismus ein Spannschloss mit zwei axial zueinander angeordneten, gegensinnigen Schraubgewinden auf. Die Verwendung der T-förmigen Steckbolzen in Verbindung mit den U-förmigen Profilen der vorhergehenden Ausgestaltungen hat sich als eine sehr kostengünstige und gleichzeitig sehr robuste Realisierung für die Kopplung der Rahmengestelle erwiesen. Des Weiteren können die Eckstücke und Steckbolzen in vorteilhafter Weise auch zur Befestigung der jeweils untersten Rahmengestelle auf der Grundplatte verwendet werden, wodurch die Teilevielfalt weiter reduziert ist.

In einer weiteren Ausgestaltung sind die Eckstücke aus einem ersten Material hergestellt und die Steckbolzen sind aus einem zweiten Material hergestellt, das weicher ist als das erste Material.

Diese Ausgestaltung besitzt den Vorteil, dass die Steckbolzen gewissermaßen als Verschleißteil fungieren. Die hohen Belastungen, denen die Eckstücke und Steckbolzen im rauen militärischen und/oder humanitären Einsatz ausgesetzt sind, wirken sich also vor allem auf die Steckbolzen aus. Demgegenüber werden die Eckstücke geschont, was vorteilhaft ist, da die Steckbolzen einfacher ausgetauscht werden können als die Eckstücke.

In einer weiteren Ausgestaltung weisen die Rahmengestelle erste Kantenprofile und zweite Kantenprofile auf, die sich von den ersten Kantenprofilen unterscheiden, wobei die ersten und zweiten Kantenprofile jeweils eine Hohlkammer und jeweils zwei vom Lagervolumen wegzeigende L-Kanten besitzen, die sich jeweils parallel zum Kantenprofil erstrecken, und wobei die ersten Kantenprofile zusätzlich einen in das Lagervolumen hineinragenden Stützbalken aufweisen, der eine weitere L-Kante parallel zum Kantenprofil ausbildet.

Es hat sich gezeigt, dass die Verwendung von zwei unterschiedlichen Kantenprofilen bei dem hier vorgesehenen Einsatzzweck der Rahmengestelle eine sehr kostengünstige Realisierung ermöglicht, obwohl durch die zwei verschiedenen Kantenprofile zunächst die Teilevielfalt erhöht wird. Der in das Lagervolumen hineinragende Stützbalken und die zusätzliche L-Kante der ersten Kantenprofile ermöglicht eine einfache Befestigung von Querstreben und/oder Bodenplatten, die ein hohes Gewicht der Aggregate, Zubehörteile oder Betriebsstoffe aufnehmen können. Daher eignen sich die ersten Kantenprofile vor allem als horizontale Kantenprofile in den Rahmengestellen der Haltekonstruktion. Für die in diesem Fall vertikal angeordneten zweiten Kantenprofile kann man hingegen vorteilhaft auf den Stützbalken verzichten, um bei voller Stabilität Material und Gewicht zu sparen und das Lagervolumen der Rahmengestelle möglichst groß zu realisieren.

In einer weiteren Ausgestaltung weisen die Rahmengestelle jeweils eine geschlossene Bodenplatte und eine geschlossene Deckplatte gegenüber von der Bodenplatte auf. In den bevorzugten Ausführungsbeispielen besitzen die Rahmengestelle zudem geschlossene Seitenwände, die je nach Anwendungsfall Klappen und/oder Türen beinhalten können. In einigen Ausführungsbeispielen sind die Bodenplatte, Deckplatte und Seitenwände mit Dämmmaterial ausgebildet, um insbesondere flüssige Betriebsstoffe und/oder temperaturempfindliche Zubehörteile und Aggregate vor extremen Temperaturänderungen zu schützen.

Die Realisierung der Rahmengestelle mit eigenen Bodenplatten und Deckplatten schützt die in den Rahmengestellen angeordneten Aggregate, Zubehörteile und Betriebsstoffe gegen Beschädigungen beim Transport und Einsatz. In einigen vorteilhaften Ausführungsbeispielen können auf den Deckplatten Arbeitsplattformen realisiert werden, beispielsweise mit Hilfe von einsteckbaren Geländerteilen. Eine dadurch gebildete, erhöhte Arbeitsplattform ist gerade bei der Dekontamination von Großgeräten, wie etwa Fahrzeugen, von großem Vorteil. Auch die Verwendung von Seitenwänden an den einzelnen Rahmengestellen schützt die untergebrachten Aggregate, Zubehörteile und Betriebsstoffe vor Umwelteinflüssen und Beschädigungen beim Transport und Einsatz.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel des neuen Gerätesystems mit zwei unterschiedlichen Typen von Rahmengestellen und einer optionalen Arbeitsplattform über einigen der Rahmengestelle,
- Fig. 2: eine zum Teil vereinfachte Darstellung eines Ausführungsbeispiels mit einem gegenüber dem Ausführungsbeispiel in Fig. 1 veränderten Funktionsumfang,
- Fig. 3: ein erstes und ein zweites Rahmengestell, die zusammen in dem Ausführungsbeispiel gemäß Fig. 2 verwendet sind,
- Fig. 4: ein Eckstück der Rahmengestelle aus Fig. 3,
- Fig. 5: einen Steckbolzen zur Kopplung zweier Eckstücke aus Fig. 4,
- Fig. 6: ein erstes Kantenprofil für die Rahmengestelle gemäß Fig. 3,
- Fig. 7: ein zweites Kantenprofil für die Rahmengestelle gemäß Fig. 3,
- Fig. 8: ein weiteres Rahmengestell mit doppelter Breite und geringer Höhe, und
- Fig. 9: ein weiteres Rahmengestell mit einfacher Breite und geringer Höhe sowie mit Boden- und Seitenwänden und einer klappbaren Deckplatte.

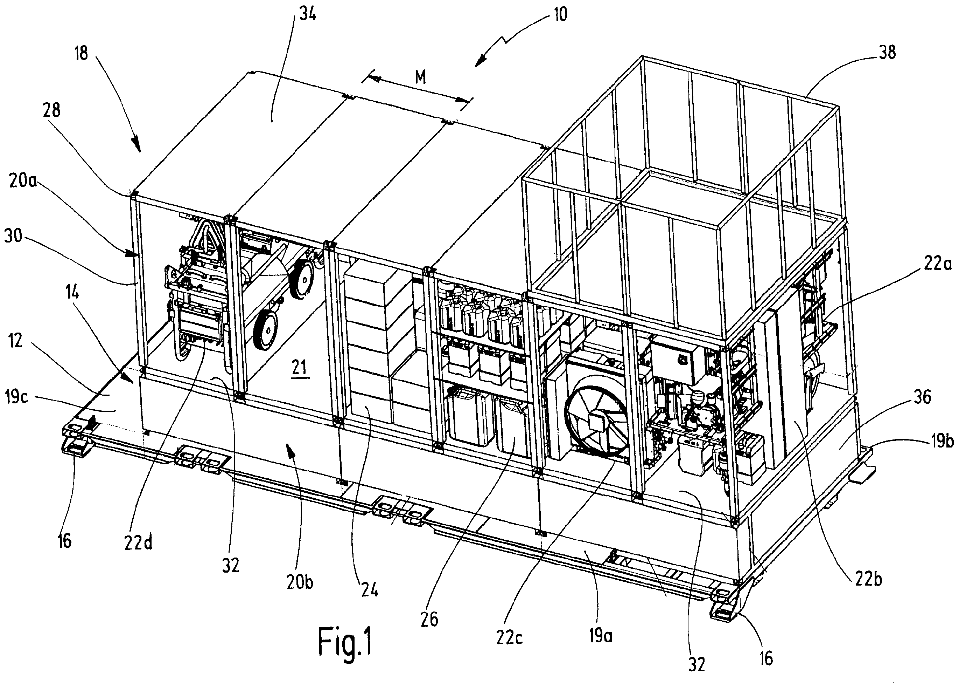

In Fig. 1 ist ein Ausführungsbeispiel des neuen Gerätesystems in seiner Gesamtheit mit der Bezugsziffer 10 bezeichnet. Das Gerätesystem 10 ist hier ein Dekontaminationssystem, das zum Entstrahlen, Entseuchen und Entgiften von Fahrzeugen, Geräten, Ausrüstungsgegenständen und/oder Personen (hier nicht dargestellt) ausgebildet ist. Ein solches Dekontaminationssystem ist ein besonders bevorzugtes Ausführungsbeispiel. Prinzipiell kann der Funktionsumfang des neuen Gerätesystems jedoch auch andere Anwendungen betreffen oder umfassen. Beispielsweise kann das neue Gerätesystem eine Anlage zur Reinigung von Schmutzwasser und zur Bereitstellung von Trinkwasser beinhalten. In den bevorzugten Ausführungsbeispielen ist das Gerätesystem mobil in dem Sinne, dass es mit Hilfe von geeigneten Transportfahrzeugen und/oder Hubschraubern als kompakte und in sich geschlossene Einheit an seinen Einsatzort gebracht werden kann.

Das Gerätesystem 10 besitzt eine Grundplatte 12 mit einer definierten Stellfläche 14 und mit Ankerelementen 16, die in den bevorzugten Ausführungsbeispielen eine lösbare Verankerung an einem Lastkraftwagen (hier nicht dargestellt) ermöglichen. In einigen Ausführungsbeispielen ist die Grundplatte ein sogenanntes Flatrack, wie es zum Transport von sperrigen Gütern im Seeverkehr verwendet wird, oder die Bodenplatte eines sogenannten ISO-Containers nach der Norm ISO 668. Derartige Flat Racks und Container besitzen Ankerelemente 16, die eine lösbare Verankerung auf der Plattform eines entsprechend ausgebildeten Lastkraftwagens ermöglichen.

Auf der Stellfläche 14 der Grundplatte 12 ist eine Haltekonstruktion 18 angeordnet, die in der nachfolgend näher beschriebenen Weise aufgebaut ist. In bevorzugten Ausführungsbeispielen ist die Haltekonstruktion 18 im zentralen Bereich der Stellfläche 14 angeordnet, so dass an zumindest zwei einander gegenüberliegenden Seiten der Haltekonstruktion 18 ein freier Randbereich 19a, 19b auf der Grundplatte 12 verbleibt. In dem dargestellten Ausführungsbeispiel der Fig. 1 verbleibt außerdem ein freier Randbereich 19c quer zu den Randbereichen 19a, 19b. Diese Randbereiche sind vorteilhaft, wenn die Haltekonstruktion in einem ISO-Container angeordnet ist, dessen Seitenwände geöffnet werden können, da sie es möglich machen, auch bei geschlossenen Seitenwänden an die Haltekonstruktion zu kommen.

Die Haltekonstruktion 18 besteht hier aus insgesamt neun Rahmengestellen 20. Dabei beinhaltet die Haltekonstruktion 18 in diesem Fall sechs erste Rahmengestelle 20a und drei zweite Rahmengestelle 20b. Die zweiten Rahmengestelle 20b sind auf der Grundplatte 12 nebeneinander angeordnet und jeweils an der Grundplatte 12 befestigt (wird weiter unten näher ausgeführt). Die ersten Rahmengestelle 20a sind auf den zweiten Rahmengestellen 20b angeordnet und an den zweiten Rahmengestellen 20b befestigt. Wie man in der Darstellung der Fig. 1 erkennen kann, besitzen die zweiten Rahmengestelle 20b in diesem bevorzugten Ausführungsbeispiel jeweils die doppelte Breite der ersten Rahmengestelle 20a und dieselbe Tiefe wie die ersten Rahmengestelle 20a. Daher kann ein zweites Rahmengestell 20b genau zwei erste Rahmengestelle 20a aufnehmen. Mit anderen Worten ist die Aufstandsfläche der zweiten Rahmengestelle 20b hier etwa doppelt so groß wie die Aufstandsfläche der ersten Rahmengestelle 20a. Andererseits besitzen die zweiten Rahmengestelle 20b in diesem Ausführungsbeispiel nur etwa ein Drittel der Höhe der ersten Rahmengestelle 20a. Das Gerätesystem 10 kommt daher mit zwei verschiedenen Typen von jeweils strukturgleichen Rahmengestellen aus, was für viele Ausführungsbespiele bevorzugt ist. Prinzipiell ist es jedoch denkbar, in anderen Ausführungsbeispielen weitere Rahmengestelle mit anderen Abmessungen vorzusehen.

Die Rahmengestelle 20a, 20b bilden jeweils ein definiertes Lagervolumen 21 aus. Die Lagervolumina 21 entsprechen dem von jedem Rahmengestell 20 umgebenen Innenraum.

Wie in dem Ausführungsbeispiel gemäß Fig. 1 erkennbar ist, sind die Lagervolumina 21 der Rahmengestelle 20 aufeinander und nebeneinander angeordnet, so dass sich im zentralen Bereich der Stellfläche 14 ein aus mehreren separaten Lagervolumina 21 gebildetes Gesamtvolumen zur Aufnahme von Gegenständen ergibt, die den Funktionsumfang des Gerätesystems 10 bestimmen.

In allen bevorzugten Ausführungsbeispielen beinhalten diese Gegenstände elektrisch betriebene Aggregate, wie Pumpen, elektrische Steuerungen, elektrische Heizungen und weitere Komponenten, wie Filter, Schlauchleitungen, Vakuumkammern und anderes. In einigen Ausführungsbeispielen beinhalten die Gegenstände ferner kraftstoffbetriebene Aggregate, wie etwa einen Diesel- oder Vielstoffbrenner zur Erzeugung von Heißdampf und/oder als Generator zur Erzeugung von Strom. In Fig. 1 sind beispielhaft ein Heißwasserhochdruckreiniger 22a inklusive einer elektrischen Steuer- und Bedieneinheit und Chemietanks 22b sowie ein (Diesel-)Generator 22c zur Stromerzeugung und ein Hochdruckreiniger 22d dargestellt, wobei der Hochdruckreiniger 22d während des Transports des Gerätesystems 10 in einem Rahmengestell 20a untergebracht ist und für den Einsatz vor Ort aus dem Rahmengestell 20a herausgenommen werden kann.

In den zweiten Rahmengestellen 20b sind hier große Flüssigkeitstanks (nicht sichtbar) untergebracht. Die Flüssigkeitstanks dienen einerseits zum Transport von Frischwasser, das für die Dekontamination von Personen oder Gegenständen benötigt wird, und sie können andererseits Schmutzwasser aufnehmen.

Des Weiteren sind in den Rahmengestellen 20a eine Vielzahl von Zubehörteilen 24 und verschiedene Betriebsstoffe 26 untergebracht. Die Betriebsstoffe 26 beinhalten beispielsweise Reinigungs- und Dekontaminationsmittel, Brennstoffe für den Diesel- oder Vielstoffbrenner oder Schmierstoffe. Die Zubehörteile 24 können diverse Kleinteile beinhalten, die für den Einsatz des Gerätesystems benötigt werden, wie etwa Sprühlanzen oder Ersatzteile für die Aggregate. Des Weiteren kann das Gerätesystem 10 Zelte, Duschwannen oder Schutzanzüge für die Bedienpersonen beinhalten (hier nicht dargestellt).

Zusammen bestimmen die Aggregate, Zubehörteile und Betriebsstoffe den Funktionsumfang des Gerätesystems 10. Dieser Funktionsumfang wird in der Regel vom Anwender, der ein solches Gerätesystem kauft, vorgegeben und der Hersteller des Gerätesystems muss die für den vorgegebenen Funktionsumfang benötigten Aggregate, Zubehörteile und Betriebsstoffe in einer für den Einsatz und Transport geeigneten Weise bereitstellen. Hierbei hilft die neue Haltekonstruktion 18 des Gerätesystems 10.

Die strukturgleichen Rahmengestelle 20 besitzen hier jeweils acht Eckstücke 28 und zwölf Kantenprofile 30. Die acht Eckstücke 28 und zwölf Kantenprofile 30 bilden zusammen jeweils ein Rahmengestell 20, das so stabil ist, dass es die darin jeweils untergebrachten Aggregate, Zubehörteile und Betriebsstoffe tragen kann. In den bevorzugten Ausführungsbeispielen sind zumindest einige Aggregate 22a, 22b, 22c stationär in den jeweiligen Rahmengestellen angeordnet und befestigt. Dies bedeutet, dass die genannten Aggregate am Einsatzort in den Rahmengestellen 20 verbleiben und von dort aus genutzt werden. Bei der Herstellung des Gerätesystems 10 können die entsprechenden Aggregate daher zunächst in den Rahmengestellen montiert und anschließend zusammengefügt werden. Die Rahmengestelle 20 bilden dabei eine universelle Haltekonstruktion, die aufgrund ihres hier einheitlichen Rastermaßes M eine Zusammenstellung des Gerätesystems 10 "nach dem Baukastenprinzip" erleichtert.

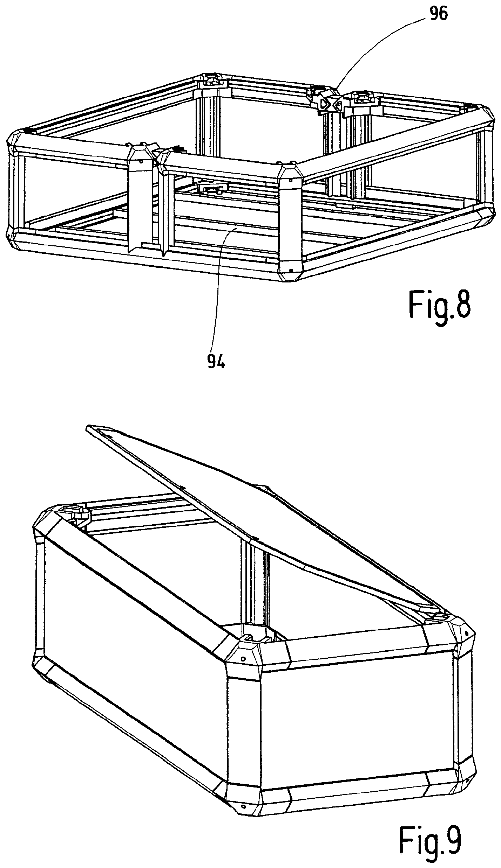

In einigen bevorzugten Ausführungsbeispielen besitzt jedes Rahmengestell 20 eine stabile Bodenplatte 32, auf der die Aggregate, Zubehörteile und/oder Betriebsstoffe gehalten sind, sowie eine Deckplatte 34 gegenüber von der Bodenplatte 32. Des Weiteren können einzelne oder alle Rahmengestelle eigene Seitenwände 36 besitzen (siehe Fig. 9). In den bevorzugten Ausführungsbeispielen sind die Eckstücke 28 und Kantenprofile 30 so ausgebildet, dass die Bodenplatte, Deckplatte und Seitenwände 36 direkt an den Kantenprofilen 30 befestigt werden können. In einigen Ausführungsbeispielen sind im Bereich der Bodenplatte 32 separate Verstärkungsstreben (siehe Fig. 8, Bezugsziffer 94) eingeschweißt und/oder eingeschraubt, um das Tragvermögen der Bodenplatte 32 zu erhöhen. In einigen Fällen sind Aggregate an den tragenden Querstreben direkt befestigt. Eine Bodenplatte 32 kann in diesen Fällen vor allem zur thermischen Isolierung ergänzend vorgesehen sein.

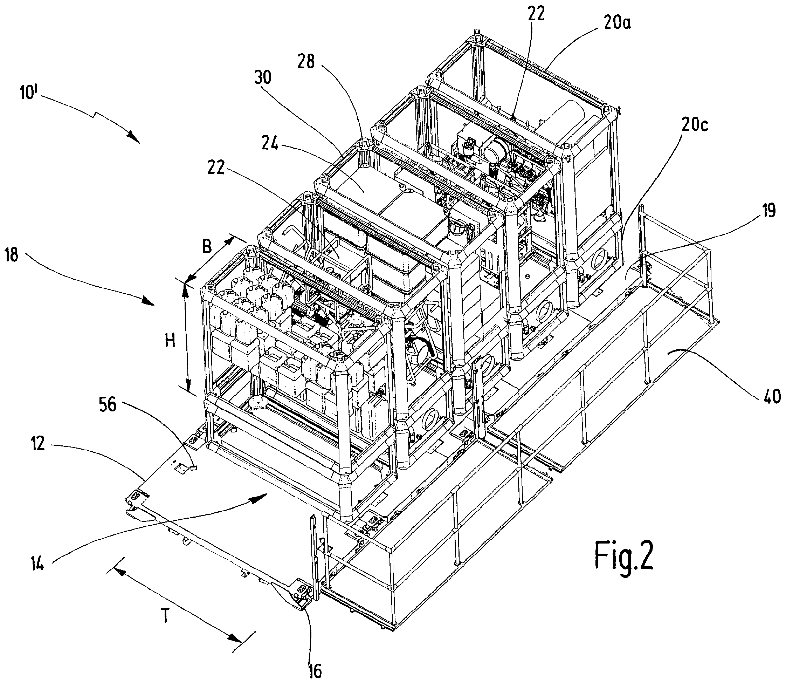

In einigen Ausführungsbeispielen besitzt das Gerätesystem eine Arbeitsplattform am Rand der Grundplatte 12, von der aus Anwender das Gerätesystem bedienen können (vgl. Fig. 2). Alternativ oder ergänzend kann das Gerätesystem 10 eine erhöhte Arbeitsplattform aufweisen, die beispielsweise das Dekontaminieren von Panzern oder anderem Großgerät erleichtert. Im vorliegenden Ausführungsbeispiel wird eine solche erhöhte Arbeitsplattform mit Hilfe eines Geländers 38 realisiert. Das Geländer 38 ist in den bevorzugten Ausführungsbeispielen ein Zubehörteil, das beim Transport des Gerätesystems 10 in einem oder mehreren der Rahmengestelle 20a oder 20b untergebracht wird und das am Einsatzort an den Eckstücken 28 und/oder Kantenprofilen 30 der ausgewählten Rahmengestelle 20a befestigt wird.

Fig. 2 zeigt ein weiteres Ausführungsbeispiel des Gerätesystems 10, hier als Gerätesystem 10' bezeichnet. Zur besseren Übersichtlichkeit sind Bodenplatten, Deckplatten und Seitenwände in der Darstellung in Fig. 2 weggelassen. Im Übrigen bezeichnen gleiche Bezugszeichen dieselben Elemente wie zuvor.

Das Gerätesystem 10' besitzt eine Haltekonstruktion 18, die von fünf ersten Rahmengestellten 20a und fünf weiteren Rahmengestellen 20c gebildet wird. Die Rahmengestelle 20a, 20c besitzen ein einheitliches Rastermaß, wobei in diesem Fall alle Rahmengestelle 20a, 20c dieselbe Breite B und dieselbe Tiefe T haben. Damit haben alle Rahmengestelle 20a, 20c in diesem Ausführungsbeispiel dieselbe Aufstandsfläche, die sich aus dem Produkt der Breite B und der Tiefe T ergibt. Die ersten Rahmengestelle 20a haben eine Höhe H, die hier etwa das Dreifache der entsprechenden Höhe der weiteren Rahmengestelle 20c ist. Vorteilhaft sind auch bei diesem Ausführungsbeispiel Flüssigkeitstanks in den Rahmengestellen 20c angeordnet. In den Rahmengestellen 20a sind Aggregate, Zubehörteile und Betriebsstoffe angeordnet und stationär befestigt, soweit dies vorteilhaft ist. Die Rahmengestelle 20a, 20c besitzen jeweils gleiche Eckstücke 28 und Kantenprofile 30, die im Folgenden näher erläutert sind.

Gemäß einem vorteilhaften Ausführungsbeispiel besitzt das Gerätesystem 10' eine Arbeitsplattform 40, die auf Höhe der Grundplatte 12 und parallel zu der Stellfläche 14 an der Grundplatte 12 befestigt werden kann. Zusammen mit dem Randbereich 19 bildet die Arbeitsplattform 40 eine Fläche, auf der ein Bediener bequem stehen kann, um an die Aggregate, Zubehörteile und/oder Betriebsstoffe heranzukommen. Die Arbeitsplattform 40 ist besonders vorteilhaft, wenn das Gerätesystem 10' auf einem Lastkraftwagen befestigt ist und von dort aus eingesetzt wird.

In bevorzugten Ausführungsbeispielen ist die Breite B der ersten Rahmengestelle 20a etwa 90 cm (ca. 3 Fuß). Die lichte Innenbreite ist vorzugsweise 80cm. Die Tiefe beträgt etwa 200 cm (ca. 7 Fuß). Die Höhe der ersten Rahmengestelle 20a beträgt in vorteilhaften Ausführungsbeispielen etwa 150 cm (5 Fuß). Diese Abmessungen haben sich als sehr vorteilhaft erwiesen, um mit den Rahmengestellen 20a, 20b, 20c Gerätesysteme aufzubauen, die mit etablierten und bewährten Transportmitteln, insbesondere mit Lastwagen und Hubschraubern, befördert werden können. In bevorzugten Ausführungsbeispielen besteht die Haltekonstruktion 18 ausschließlich aus Rahmengestellen 20a, 20b (siehe Fig. 1) und/oder 20c, d.h. die Anzahl der verwendeten Rahmengestelle ist auf zwei oder maximal drei strukturgleiche Typen beschränkt.

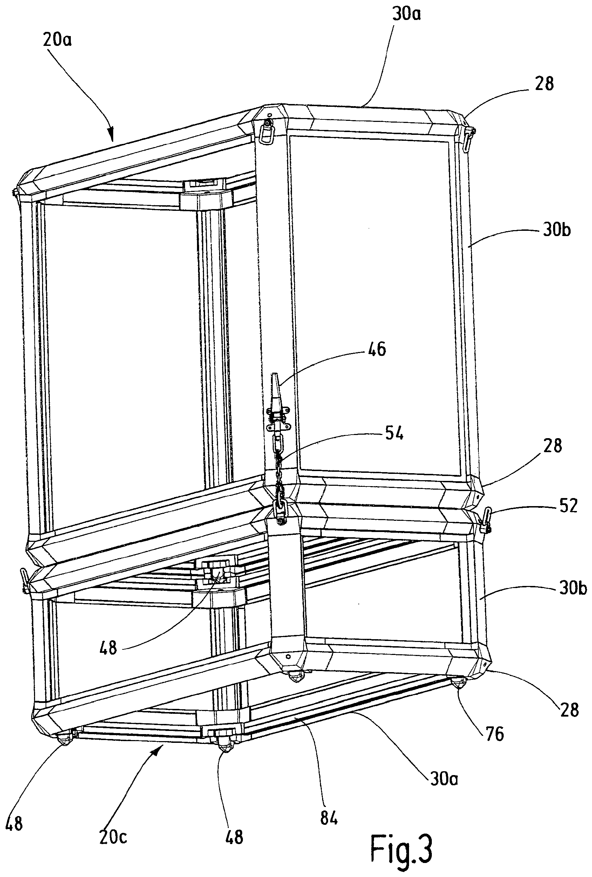

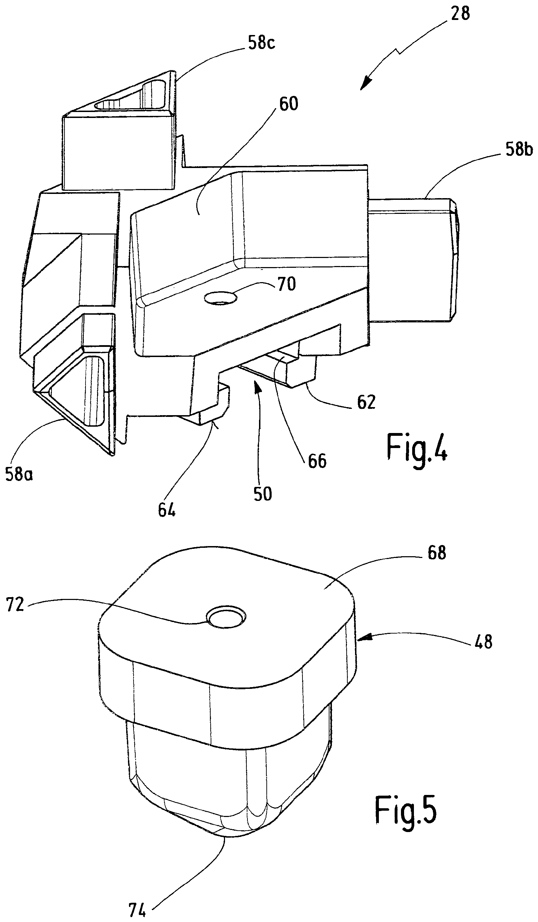

Fig. 3 zeigt eine vergrößerte Darstellung von einem ersten Rahmengestell 20a und einem weiteren Rahmengestell 20c aus dem Gerätesystem 10' der Fig. 2. Wie man erkennen kann, sind das erste Rahmengestell 20a und das weitere Rahmengestell 20c hier mit Hilfe eines Spannmechanismus 46 und mit Hilfe von Steckbolzen 48 lösbar aneinander befestigt. Die Steckbolzen 48 (siehe Fig. 5) werden in eine taschenförmige Vertiefung 50 (siehe Fig. 4) eingesteckt, die an jedem der Eckstücke 28 ausgebildet ist. Die Steckbolzen 48 sorgen in Kombination mit den Eckstücken 28 für eine sehr robuste und stabile Befestigung der Rahmengestelle 20a, 20c in zwei der drei möglichen Raumrichtungen, wobei diese zwei Raumrichtungen in den bevorzugten Ausführungsbeispielen horizontal liegen. Zur Fixierung der Rahmengestelle 20a, 20c in der dritten Raumrichtung dient der Spannmechanismus 46, der hier nur an einer Stelle dargestellt ist. In den bevorzugten Ausführungsbeispielen sind entsprechende Spannmechanismen 46 an allen vier Eckstücken 28 angeordnet, an denen zwei aufeinander angeordnete Rahmengestelle aneinander anliegen. Dementsprechend sind zwei Rahmengestelle 20a, 20c in den bevorzugten Ausführungsbeispielen über jeweils vier aneinander liegende Eckstückpaare, einen in jedes Eckstückpaar eingesteckten Steckbolzen 48 und vier Spannmechanismen 46 aneinander befestigt.

Wie in Fig. 3 dargestellt ist, besitzen die alle oberen Eckstücke 28 jeweils eine Öse 52, in die eine Kette 54 eingehängt werden kann. Die Kette 54 kann mit Hilfe des Spannmechanismus 46, der hier beispielhaft einen Exzenter beinhaltet, gespannt werden, um auf diese Weise die aufeinander angeordneten Rahmengestelle 20a, 20c in vertikaler Richtung zu fixieren. In anderen Ausführungsbeispielen beinhaltet der Spannmechanismus 46 ein Spannschloss mit zwei axial zueinander angeordneten, gegensinnigen Schraubgewinden (Rechts- und Linksgewinde). In horizontaler Richtung sorgen die Steckbolzen 48 in den Eckstücken 28 für eine stabile Befestigung.

In den bevorzugten Ausführungsbeispielen werden die Rahmengestelle 20 in gleicher Weise auf der Grundplatte 12 befestigt. Dazu sind in der Grundplatte 12 weitere Vertiefungen 56 (Fig. 2) angeordnet. Die freien Enden der Steckbolzen in den Eckstücken 28 der jeweils unten angeordneten Rahmengestelle 20b oder 20c ragen in die Löcher oder Vertiefungen der Grundplatte 12 hinein, um eine Fixierung in horizontaler Richtung zu gewährleisten. In vertikaler Richtung werden die Rahmengestelle dann mit einem Spannmechanismus 46 und einer Kette 54 an der Grundplatte 12 verspannt.

Wie man in Fig. 4 erkennen kann, sitzt jedes Eckstück hier drei orthogonal zueinander angeordnete Zapfen 58a, 58b, 58c. In den bevorzugten Ausführungsbeispielen haben die Zapfen 58 einen polygonalen Querschnitt, der insbesondere eine weitgehend dreieckige Grundform aufweist. Die Zapfen 58 sind über ein Mittelteil 60 materialschlüssig miteinander verbunden. Das Mittelteil 60 weist an einer Seite, die von einem der Zapfen (hier der Zapfen 58c) abgewandt ist, eine taschenförmige Vertiefung 50 auf, die von einem U-förmigen Profil 62 begrenzt ist. Das U-förmige Profil 62 besitzt eine plane Außenseite 64, die eine definierte Überhöhung an dem zugehörigen Eckstück bildet. Mit anderen Worten bildet die plane Außenseite 64 eine definierte Aufstandsfläche, an der zwei gegenüberliegende Eckstücke 28 plan aneinander liegen können, ohne dass die Kantenprofile 30 der jeweils zugehörigen Rahmengestelle gegeneinander stoßen. Das U-förmige Profil 62 bildet in der Vertiefung 50 einen Hinterschnitt, an dem der Kopf 68 eines Steckbolzens 48 gehalten ist. Vorteilhaft besitzt das Mittelteil eine Bohrung 70 und im Kopf des Steckbolzens 48 ist eine Gewindebohrung 72 angeordnet, die bei eingestecktem Steckbolzen 48 mit der Bohrung 70 fluchtet. Durch die Bohrung 70 kann der Steckbolzen 48 gesichert werden, indem eine Schraube (hier nicht dargestellt) durch die Bohrung 70 in die Bohrung 72 geschraubt wird. Das freie Ende 74 des Steckbolzens 48 besitzt in einigen Ausführungsbeispielen eine weitere Bohrung 76, in die durch die Vertiefung 50 des darunter liegenden Eckstücks ebenfalls eine Schraube eingeschraubt werden kann (hier nicht dargestellt).

In den bevorzugten Ausführungsbeispielen sind die Eckstücke 28 Gussstücke aus Aluminium oder einer Aluminiumlegierung mit einer Härte, die härter ist als das Material, aus dem die Steckbolzen 48 hergestellt sind. In diesem Fall wirken die Steckbolzen 48 als Verschleißteile, die gegebenenfalls nach häufigem Lösen und Verspannen von Rahmengestellen ausgetauscht werden können, ohne dass die Rahmengestelle selbst zerlegt werden müssen.

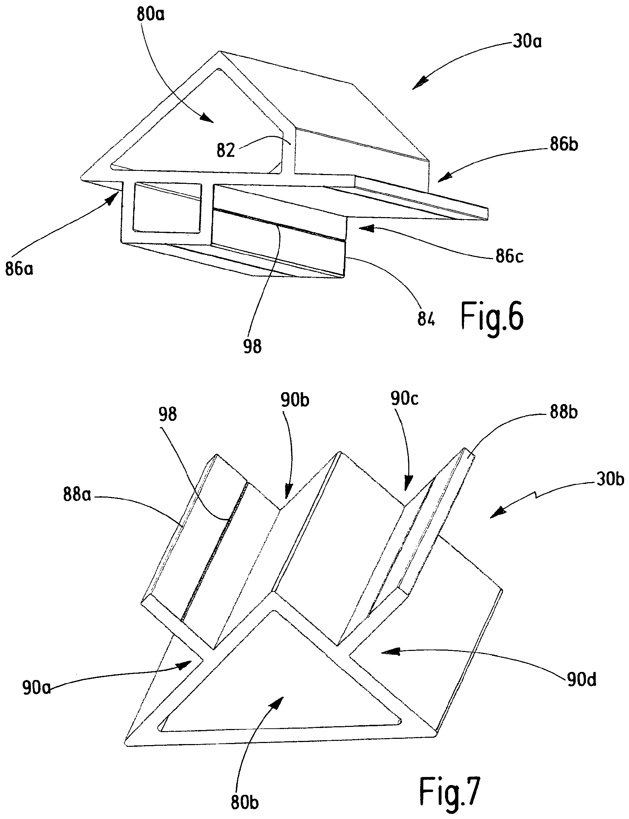

In den Fig. 6 und 7 sind vorteilhafte Kantenprofile dargestellt, die in den bevorzugten Ausführungsbeispielen auf die Zapfen 58 der Eckstücke 28 aufgesteckt werden. Fig. 6 zeigt ein Kantenprofil 30a, das in den bevorzugten Ausführungsbeispielen für die horizontal liegenden Kantenprofile verwendet wird. Fig. 7 zeigt ein weiteres Kantenprofil 30b, das in den bevorzugten Ausführungsbeispielen für die vertikalen Kantenprofile verwendet wird. Wie man erkennen kann, sind die Kantenprofile 30a, 30b verschieden voneinander.

Jedes der Kantenprofile 30a, 30b besitzt eine Hohlkammer 80. Bei dem Kantenprofil 30b besitzt die Hohlkammer 80b einen dreieckigen Querschnitt, so dass das Kantenprofil 30b auf den im Querschnitt dreieckigen Zapfen 58c passgenau aufgesetzt werden kann. Die Hohlkammer 80a des Kantenprofils 30a besitzt einen weitgehend dreieckigen Querschnitt, allerdings mit einer geraden Kante 82 anstelle einer dritten Spitze. Daher hat die Hohlkammer 80a hier einen polygonalen Querschnitt mit vier Ecken, wobei die Grundform derjenigen eines Dreiecks angenähert ist. Diese Form hat sich als vorteilhaft erwiesen, weil sie eine hohe Stabilität bei relativ geringem Gewicht ermöglicht.

Das Kantenprofil 30a besitzt ferner einen integrierten Stützbalken 84, der bei den Rahmengestellen 20 zum Lagervolumen 21 zeigt (siehe Fig. 3). Der Stützbalken 84 ermöglicht eine einfache Befestigung von Verstärkungsstreben (hier nicht dargestellt), was insbesondere im Bereich der Bodenplatte 32 und der Deckplatte 34 von Vorteil ist.

Darüber hinaus bildet jedes Kantenprofil 30a drei L-förmige Kanten 86a, 86b, 86c aus. Die L-Kanten 86a, 86b zeigen mit ihrer "Öffnung" vom Lagervolumen 21 weg und sie bilden jeweils eine Anlage- und Montagefläche für die die Bodenplatte 32, Deckplatte 34 und/oder Seitenwände 36. Die dritte L-Kante 86c zeigt zum Lagervolumen 21 hin. Hier kann vorteilhaft eine Bodenplatte oberhalb von Stützstreben (hier nicht dargestellt) montiert werden, die dann durch die Stützstreben zusätzliche Stabilisierung erfährt.

Die vertikalen Kantenprofile 30b unterscheiden sich von den horizontalen Kantenprofilen 30a vor allem dadurch, dass der Stützbalken 84 entfällt. Stattdessen besitzen die Kantenprofile 30b hier zwei Flügel 88a, 88b, die insgesamt vier L-Kanten 90a bis 90d ausbilden. Prinzipiell würde es genügen, wenn die vertikalen Kantenprofile 30b lediglich zwei L-Kanten 90a, 90d ausbilden, die wiederum als Monateflächen zur Befestigung der Seitenwände dienen. Mit den Flügeln 88 lassen sich die zwei vorteilhaften L-Kanten 90a, 90d mit geringem Materialgewicht realisieren. Außerdem können in den innenliegenden Kanten 90b und 90c vorteilhaft Einbauten im Inneren der Rahmengestelle, bspw. Aggregate und/oder Zubehörteile, befestigt werden. Um die Montage von Querstreben, Einbauten und/oder Deck-, Boden- und/oder Seitenplatten zu erleichtern, weisen der Stützbalken 84 und die Flügel 88 jeweils Anreißlinien 98 auf, die in den bevorzugten Ausführungsbeispielen als durchgehende linienförmige Vertiefung ausgebildet sind.

In allen bevorzugten Ausführungsbeispielen sind die Kantenprofile 30a, 30b Alustranggussprofile. Die Bodenplatten, Deckplatten und Seitenwände sind Sandwichplatten mit einem Kunststoffkern, der von zwei Aluminium- oder Glasfaserkaschierungen bedeckt ist. Vorteilhaft sind die Sandwichplatten in den L-Kanten 86, 90 der Kantenprofile 30 verschraubt und/oder verklebt.

Wie man in Fig. 4 erkennen kann, sind die Eckstücke 28 im Bereich der Zapfen 58 mit zahlreichen zueinander schrägen Flächen ausgebildet, was das Entformen der Eckstücke 28 aus den Gussformen (hier nicht dargestellt) begünstigt. Andererseits erschweren die schrägen Außenseiten der Eckstücke 28 eine seitliche Verbindung von zwei Eckstücken 28 abseits der U-förmigen Profile 62. Daher können in einigen Ausführungsbeispielen Keilstücke (siehe Fig. 8, Bezugsziffer 96) zwischen zwei benachbarte Eckstücke eingeschweißt sein, um auf diese kostengünstige Weise eine "Doppelecke" mit sechs Zapfen zu realisieren. Mit der Doppelecke können stabile Rahmengestelle mit doppelter oder dreifacher Breite des Rastermaßes sehr kostengünstig realisiert werden. In den bevorzugten Ausführungsbeispielen sind die nebeneinander angeordnete Rahmengestelle jedoch nicht aneinander befestigt, sondern mit einem seitlichen Abstand von ca. 20 bis 30 mm angeordnet. In einigen Ausführungsbeispielen können Gummipuffer zwischen nebeneinander angeordnete Rahmengestelle platziert sein, um Stoß- oder Schlaggeräusche zu unterbinden.

Ein Vorteil der bevorzugten Rahmengestelle mit den Eckstücken 28 liegt darin, dass die Eckstücke 28 lediglich über die Steckbolzen und den Spannmechanismus miteinander gekoppelt werden. Die bevorzugten Rahmengestelle verzichten daher auf bewegliche Teile zur gegenseitigen Kopplung.

## Patentansprüche

1. Gerätesystem für militärische und/oder humanitäre Operationen, insbesondere mobiles Dekontaminationssystem, mit einer Vielzahl an elektrischen und/oder kraftstoffbetriebenen Aggregaten (22), Zubehörteilen (24) und Betriebsstoffen (26), die zusammen einen Funktionsumfang des Gerätesystems bestimmen, wobei die elektrischen und/oder kraftstoffbetriebenen Aggregate (22) zumindest eine der folgenden Komponenten beinhalten: einen Generator zur Erzeugung von elektrischem Strom, eine oder mehrere Pumpen zur Umwälzung, Förderung und/oder Austragung von Flüssigkeiten, eine oder mehrere Pumpen zur Erzeugung von Unterdruck, einen Brenner und/oder eine elektrische Heizung zur Erhitzung von auszutragenden Flüssigkeiten, Dekontaminationsmitteln und/oder anderen Chemikalien, Filter, Prozesssteuergeräte und ein Bedienpult, ferner mit einer lasttragenden Grundplatte (12) mit einer definierten Stellfläche (14) und mit Ankerelementen (16), die eine lösbare Verankerung an einem Transportmittel, insbesondere an einem Transportfahrzeug, ermöglichen, und mit einer Haltekonstruktion (18), die auf der Stellfläche (14) befestigt ist und die dazu ausgebildet ist, die Aggregate (22) beim Transport und im Betrieb auf der Grundplatte (12) zu halten, **dadurch gekennzeichnet, dass** die Haltekonstruktion (18) aus einer Vielzahl von selbsttragenden, strukturgleichen und jeweils quaderförmigen Rahmengestellen (20) gebildet ist, die nebeneinander und/oder aufeinander angeordnet und an der Grundplatte (12) befestigt sind, **dadurch gekennzeichnet, dass** die Rahmengestelle (20) jeweils acht Eckstücke (28) und zwölf Kantenprofile (30) besitzen, die zusammen jeweils ein definiertes Lagervolumen (21) umgeben, und wobei die Aggregate (22), Zubehörteile (24) und Betriebsstoffe (26) durchweg in den definierten Lagervolumina (21) der Rahmengestelle (20) angeordnet sind.

2. Gerätesystem nach Anspruch 1, **dadurch gekennzeichnet, dass** die Rahmengestelle (20) einen zentralen Bereich der Stellfläche (14) belegen, so dass auf der Grundplatte (12) an zumindest zwei Seiten ein freier Randbereich (19) verbleibt.

3. Gerätesystem nach Anspruch 2, **gekennzeichnet durch** zumindest eine mobile Arbeitsplattform (40), die auf Höhe der Grundplatte (12) an dem Randbereich (19) positionierbar ist.

4. Gerätesystem nach einem der Ansprüche 1 bis 3, **dadurch gekennzeichnet, dass** die elektrischen und/oder kraftstoffbetriebenen Aggregate (22) in den jeweiligen Rahmengestellen (20) stationär montiert sind.

5. Gerätesystem nach einem der Ansprüche 1 bis 4, **dadurch gekennzeichnet, dass** Rahmengestelle (20) ein einheitliches Rastermaß in Bezug auf Breite, Tiefe und/oder Höhe aufweisen.

6. Gerätesystem nach einem der Ansprüche 1 bis 5, **dadurch gekennzeichnet, dass** die Vielzahl der Rahmengestelle (20) erste Rahmengestelle (20a) und zweite Rahmengestelle (20b) beinhaltet, wobei die ersten Rahmengestelle (20a) eine erste Aufstandsfläche mit einer ersten Breite und einer ersten Tiefe sowie eine erste Höhe haben, wobei die zweiten Rahmengestelle (20b) eine zweite Aufstandsfläche mit einer zweiten Breite und einer zweiten Tiefe sowie eine zweite Höhe haben, wobei die zweite Tiefe gleich der ersten Tiefe ist, und wobei die zweite Höhe etwa ein Drittel der ersten Höhe ist.

7. Gerätesystem nach Anspruch 6, **dadurch gekennzeichnet, dass** die zweite Breite das Doppelte der ersten Breite ist.

8. Gerätesystem nach Anspruch 6 oder 7, **dadurch gekennzeichnet, dass** die erste Breite etwa 90 cm beträgt und dass die erste Tiefe etwa 200 cm beträgt.

9. Gerätesystem nach einem der Ansprüche 1 bis 8, **gekennzeichnet durch** zumindest einen Flüssigkeitstank, der in einem der Rahmengestelle (20) dauerhaft befestigt ist.

10. Gerätesystem nach einem der Ansprüche 1 bis 9, **dadurch gekennzeichnet, dass** die Eckstücke (28) jeweils drei orthogonal zueinander angeordnete Zapfen (58) aufweisen, wobei jeder Zapfen (58) mit jeweils einem Kantenprofil (30) dauerhaft verbunden ist.

11. Gerätesystem nach einem der Ansprüche 1 bis 10, **dadurch gekennzeichnet, dass** die Eckstücke (28) jeweils eine taschenförmige Vertiefung (50) aufweisen, die an einer Seite von einem U-förmigen Profil (62) begrenzt ist, welches in der Vertiefung (50) einen Hinterschnitt (66) bildet.

12. Gerätesystem nach Anspruch 11, **dadurch gekennzeichnet, dass** das U-förmige Profil (62) eine plane Außenseite (64) aufweist, die eine definierte Überhöhung an dem zugehörigen Eckstück (28) bildet.

13. Gerätesystem nach einem der Ansprüche 1 bis 12, **gekennzeichnet durch** eine Vielzahl T-förmiger Steckbolzen (48), mit denen aneinander liegende Eckstücke (28) von zwei übereinander angeordneten Rahmengestellen (20) mechanisch verbunden sind.

14. Gerätesystem nach Anspruch 13, **dadurch gekennzeichnet, dass** die Eckstücke (28) aus einem ersten Material hergestellt sind und dass die Steckbolzen (48) aus einem zweiten Material hergestellt sind, das weicher als das erste Material ist.

15. Gerätesystem nach einem der Ansprüche 1 bis 14, **dadurch gekennzeichnet, dass** die Rahmengestelle (20) erste Kantenprofile (30a) und zweite Kantenprofile (30b) aufweisen, die sich von den ersten Kantenprofilen (30a) unterscheiden, wobei die ersten und zweiten Kantenprofile (30a, 30b) jeweils eine Hohlkammer (80) und jeweils zwei vom Lagervolumen weg zeigende L-Kanten (86, 90) besitzen, die sich jeweils parallel zum Kantenprofil erstrecken, und wobei die ersten Kantenprofile (30a) zusätzlich einen in das Lagervolumen (21) hineinragenden Stützbalken (84) aufweisen, der eine weitere L-Kante (86c) parallel zum Kantenprofil ausbildet.

## Claims

1. A device system for military and/or humanitarian operations, in particular a mobile decontamination system, comprising a plurality of electrical and/or fuel-operated units (22), accessory parts (24) and operating supplies (26), which together determine a functional scope of the device system, with the electrical and/or fuel-operated units (22) the units include at least one of the following components: a generator for generating electrical power, one or more pumps for recirculating, conveying and/or discharging liquids, one or more pumps for generating negative pressure, a burner and/or an electrical heater for heating liquids, decontaminants and/or other chemicals to be discharged, filters, process control devices and a control panel, and comprising a load-bearing base plate (12) having a defined placement surface (14) and anchoring elements (16), which enable detachable anchoring to a transport means, in particular to a transport vehicle, and comprising a retaining structure (18), which is fastened on the placement surface (14) and which is designed to hold the units (22) on the base plate (12) during transport and during operation, **characterized in that** the retaining structure (18) is formed from a plurality of self-supporting, structurally identical, cuboid-shaped frames (20), which are arranged next to each other and/or on top of each other and are fastened to the base plate (12), **characterized in that** the frames (20) each have eight corner pieces (28) and twelve edge profile elements (30), which together enclose a defined storage volume (21), and wherein the units (22), accessory parts (24) and operating supplies (26) are consistently arranged in the defined storage volumes (21) of the frames (20).

2. The device system of claim 1, **characterized in that** the frames (20) occupy a central region of the placement surface (14), such that a free edge region (19) remains on the base plate (12) on at least two sides.

3. The device system of claim 2, **characterized by** at least one mobile working platform (40), which can be positioned at the height of the base plate (12) at the edge region (19).

4. The device system of any one of claims 1 to 3, **characterized in that** the electrical and/or fuel-operated units (22) are mounted in the respective frames (20) in a stationary manner.

5. The device system of any one of claims 1 to 4, **characterized in that** the frames (20) have a uniform modular dimension in terms of width, depth and/or height.

6. The device system of any one of claims 1 to 5, **characterized in that** the plurality of frames (20) includes first frames (20a) and second frames (20b), wherein the first frames (20a) have a first footprint having a first width and a first depth and a first height, wherein the second frames (20b) have a second footprint having a second width and a second depth and a second height, wherein the second depth is the same as the first depth, and wherein the second height is approximately a third of the first height.

7. The device system of claim 6, **characterized in that** the second width is twice the first width.

8. The device system of claim 6 or 7, **characterized in that** the first width is approximately 90 cm and the first depth is approximately 200 cm.

9. The device system of any one of claims 1 to 8, **characterized by** at least one liquid tank, which is permanently fastened in one of the frames (20).

10. The device system of any one of claims 1 to 9, **characterized in that** the corner pieces (28) each have three pins (58) arranged orthogonally to one another, wherein each pin (58) is permanently connected to an edge profile (30).

11. The device system of any one of claims 1 to 10, **characterized in that** the corner pieces (28) each have a pocket-like indentation (50), which is delimited on one side by a U-shaped profile element (62), which forms an undercut (66) in the indentation (50).

12. The device system of claim 11, **characterized in that** the U-shaped profile element (62) has a planar outer face (64), which forms a defined elevation on the associated corner piece (28).

13. The device system of any one of claims 1 to 12, **characterized by** a plurality of T-shaped lock pins (48) with which adjacently arranged corner pieces (28) of two frames (20) arranged on top of each other are mechanically connected.

14. The device system of claim 13, **characterized in that** the corner pieces (28) are produced from a first material, and the lock pins (48) are produced from a second material, which is softer than the first material.

15. The device system of any one of claims 1 to 14, **characterized in that** the frames (20) have first edge profile elements (30a) and second edge profile elements (30b), which differ from the first edge profile elements (30a), wherein the first and second edge profile elements (30a, 30b) each have a hollow chamber (80) and each have two L-edges (86, 90), which point away from the storage volume and which each extend parallel to the edge profile element, and wherein the first edge profile elements (30a) additionally have a support beam (84) protruding into the storage volume (21), which support beam forms a further L-edge (86c) parallel to the edge profile element.

## Revendications

1. Système d'appareils pour des opérations militaires et/ou humanitaires, notamment système de décontamination mobile, comprenant une pluralité de groupes (22) fonctionnant à l'électricité et/ou avec un carburant, pièces accessoires (24) et consommables (26), lesquels définissent ensemble une étendue fonctionnelle du système d'appareils, les groupes (22) fonctionnant à l'électricité et/ou avec un carburant contenant au moins l'un des composants suivants : un générateur destiné à générer un courant électrique, une ou plusieurs pompes destinées à la circulation, au refoulement et/ou à l'extraction de liquides, une ou plusieurs pompes destinées à générer une dépression, un brûleur et/ou un chauffage électrique destinés à chauffer des liquides à extraire, des moyens de décontamination et/ou d'autres produits chimiques, des filtres, des contrôleurs de processus et un pupitre de commande, comprenant en outre une plaque de base (12) porteuse de charge pourvue d'une surface d'installation (14) définie et comprenant des éléments d'ancrage (16) qui permettent un ancrage amovible à des moyens de transport, notamment à un véhicule de transport, et comprenant une construction de maintien (18) qui est fixée sur la surface d'installation (14) et qui est configurée pour maintenir les groupes (22) lors du transport et en fonctionnement sur la plaque de base (12), **caractérisé en ce que** la construction de maintien (18) est constituée d'une pluralité de bâtis (20) autoporteurs, de structure identique et respectivement parallélépipédiques, lesquels sont disposés les uns à côté des autres et/ou les uns sur les autres et sont fixés à la plaque de base (12), **caractérisé en ce que** les bâtis (20) possèdent respectivement huit pièces de coin (28) et douze profilés d'arête (30) qui, ensemble, entourent respectivement un volume de stockage (21) défini, et les groupes (22), les pièces accessoires (24) et les consommables (26) étant entièrement disposés dans les volumes de stockage (21) définis des bâtis (20).

2. Système d'appareils selon la revendication 1, **caractérisé en ce que** les bâtis (20) occupent une zone centrale de la surface d'installation (14), de sorte qu'il reste une zone de bordure (19) libre sur au moins deux côtés sur la plaque de base (12).

3. Système d'appareils selon la revendication 2, **caractérisé par** au moins une plate-forme de travail mobile (40) qui peut être positionnée à la hauteur de la plaque de base (12) sur la zone de bordure (19).

4. Système d'appareils selon l'une des revendications 1 à 3, **caractérisé en ce que** les groupes (22) fonctionnant à l'électricité et/ou avec un carburant sont montés en position fixe dans les bâtis (20) respectifs.

5. Système d'appareils selon l'une des revendications 1 à 4, **caractérisé en ce que** les bâtis (20) possèdent une dimension modulaire uniforme en référence à la largeur, la profondeur et/ou la hauteur.

6. Système d'appareils selon l'une des revendications 1 à 5, **caractérisé en ce que** la pluralité de bâtis (20) contient des premiers bâtis (20a) et des deuxièmes bâtis (20b), les premiers bâtis (20a) ayant une première surface d'appui qui présente une première largeur et une première profondeur ainsi qu'une première hauteur, les deuxièmes bâtis (20b) ayant une deuxième surface d'appui qui présente une deuxième largeur et une deuxième profondeur ainsi qu'une deuxième hauteur, la deuxième profondeur étant égale à la première profondeur et la deuxième hauteur étant égale à environ un tiers de la première hauteur.

7. Système d'appareils selon la revendication 6, **caractérisé en ce que** la deuxième largeur est égale au double de la première largeur.

8. Système d'appareils selon la revendication 6 ou 7, **caractérisé en ce que** la première largeur est égale à environ 90 cm et **en ce que** la première profondeur est égale à environ 200 cm.

9. Système d'appareils selon l'une des revendications 1 à 8, **caractérisé par** au moins un réservoir à liquide qui est fixé à demeure dans l'un des bâtis (20).

10. Système d'appareils selon l'une des revendications 1 à 9, **caractérisé en ce que** les pièces de coin (28) possèdent respectivement trois tenons (58) disposés de manière orthogonale les uns par rapport aux autres, chaque tenon (58) étant relié à demeure respectivement à un profilé d'arête (30).

11. Système d'appareils selon l'une des revendications 1 à 10, **caractérisé en ce que** les pièces de coin (28) possèdent respectivement une cavité (50) en forme de poche, laquelle est délimitée d'un côté par un profilé en forme de U (62) qui forme une contre-dépouille (66) dans la cavité (50).

12. Système d'appareils selon la revendication 11, **caractérisé en ce que** le profilé en forme de U (62) possède un côté extérieur plan (64) qui forme un surhaussement défini au niveau de la pièce de coin (28) associée.

13. Système d'appareils selon l'une des revendications 1 à 12, **caractérisé par** une pluralité d'axes embrochables en forme de T (48) avec lesquels sont reliées mécaniquement les pièces de coin (28) disposées les unes contre les autres de deux bâtis (20) superposés.

14. Système d'appareils selon la revendication 13, **caractérisé en ce que** les pièces de coin (28) sont fabriquées à partir d'un premier matériau et **en ce que** les axes embrochables (48) sont fabriqués à partir d'un deuxième matériau qui est plus tendre que le premier matériau.

15. Système d'appareils selon l'une des revendications 1 à 14, **caractérisé en ce que** les bâtis (20) présentent des premiers profilés d'arête (30a) et des deuxièmes profilés d'arête (30b), lesquels sont différents des premiers profilés d'arête (30a), les premiers et les deuxièmes profilés d'arête (30a, 30b) possédant respectivement une chambre creuse (80) et respectivement deux arêtes en L (86, 90) orientées à l'opposé du volume de stockage, lesquelles s'étendent respectivement en parallèle du profilé d'arête, et les premiers profilés d'arête (30a) présentant en plus une poutrelle support (84) qui fait saillie à l'intérieur du volume de stockage (21), laquelle forme une arête en L (86c) supplémentaire parallèle au profilé d'arête.
